# EUROPEAN PATENT APPLICATION

(11) **EP 1 488 811 A1**
(43) Date of publication of application: **22.12.2004**
(21) Application number: 03710264.7
(22) Date of filing: 06.03.2003
(51) Int. Cl.: A61K 47/10

(54) **TABLETS QUICKLY DISINTEGRATING IN ORAL CAVITY**

(30) Priority: 06.03.2002 JP 2002060922
(71) Applicant: KYOWA HAKKO KOGYO CO., LTD., Chiyoda-ku, Tokyo 100-8185 (JP)
(72) Inventor: OHTA, Motohiro, Shizuoka 411-8731 (JP); IWASE, Yuji, Shizuoka 411-8731 (JP); SAITO, Naohiro, Shizuoka 411-8731 (JP); MORIMOTO, Kiyoshi, Shizuoka 411-8731 (JP)
(74) Representative: VOSSIUS & PARTNER
(86) International application number: PCT/JP2003/002660
(87) International publication number: WO 2003/074085

(57) **Abstract**

According to the present invention, provided are
an intraorally rapidly disintegrable tablet which comprises D-mannitol and a disintegrator in addition to fine granules prepared by granulating a mixture of a water-soluble pharmacologically active ingredient and an adsorbent;
a process for producing an intraorally rapidly disintegrable tablet which comprises mixing D-mannitol and a disintegrator with fine granules prepared by granulating a mixture of a water-soluble pharmacologically active ingredient and an adsorbent to yield a material for compression molding, and subjecting the material to compression molding; and
an intraorally rapidly disintegrable tablet which is prepared by mixing D-mannitol and a disintegrator with fine granules prepared by granulating a mixture of a water-soluble pharmacologically active ingredient and an adsorbent to yield a material for compression molding, subjecting the material to compression molding.

## Description

### Technical Field

The present invention relates to intraorally rapidly disintegrable tablets.

### Background Art

As for the major formulation of orally administrable pharmaceuticals, there are solid preparations such as granules, powders or fine granules, tablets, and capsules, and liquid preparations such as syrup. Among these preparations, granules, powders or fine granules frequently give an unpleasant feeling to a person receiving such preparations, for example, a sandy feel or lodging between the teeth. Tablets or capsules are very easy to handle for recipients in comparison with granules, powders or fine granules, and widely employed as oral pharmaceutical preparations. However, these preparations are difficult to be taken by the aged or infants whose swallowing ability is weak, because those preparation sometimes stuck halfway on their esophagus. Such solid preparations are prepared in order that they are disintegrated and dissolved in the digestive organ via oral administration, and the pharmacologically active ingredient is absorbed therein; therefore, they have usually no rapid disintegration property or solubility in the oral cavity. On the other hand, syrup is an easily administrable preparation for the aged and infants, but it has a problem that the accurate measurement is hardly expected.

In this situation in pharmaceutical preparations, it has been desired to develop an intraorally rapidly disintegrable solid preparation that can easily be taken by patients including aged persons and infants whose swallowing ability is weak, anytime and anywhere with ease without water, in view of increase of the aging population and change of the living environment, with keeping the handiness, which is a convenient characteristic of tablets. Tablets that are rapidly disintegrated in the oral cavity can easily be taken by a patient whose swallowing ability is weak at an accurate dose, different from liquid preparations such as syrup. Moreover, for other patients than the aged or infants, the tablets can be taken without water in the outdoors where no water is available, and thus, the intraorally rapidly disintegrable solid preparation will improves the medication compliance for all of the patients.

A preparation which is merely rapidly disintegrable can be easily prepared by pressing and molding the pharmaceutical components at low pressure, however, such preparation shows poor pharmaceutical strength, cannot keep their shape, and sometimes results in disintegration in the course of packaging or distribution as well as during taking-out of the preparation from the package by a recipient who intends to take it. Therefore, an intraorally rapidly disintegrable preparations should have not only an excellent intraoral disintegrability but also the pharmaceutical strength with no problem in handling.

However, in general, the solubility of a tablet is antinomic to the hardness of the tablet. That is, the improvement of the solubility of tablets to increase the rate of dissolution results in deterioration of the tablet hardness. The hardness of tablets is an important factor for tablets in the course of transportation and packaging in the production process, in the course of distribution, and during taking-out of a tablet from the package by a recipient who intends to take it, as described above. If the hardness of tablets is insufficient, the tablets can not retain their shape and lose shape in the above course.

As for the known technique for producing preparations which rapidly disintegrate and dissolve in the oral cavity, a process has been proposed which comprises dissolving or suspending conventional pharmaceutical components in an aqueous solvent, filling the solution in a pre-formed pocket of blister package, and subj ecting the solution to freeze-drying or vacuum-drying for dehydration. Though these solid preparations have rapid solubility, there still remains a problem that sufficient mechanical strength is not attained and it is difficult to treat in a usual manner due to their high hygroscopicity. Further, there is another problem that much time is required in the production because the above proces s for producing the solid preparation comprises dissolving a variety of components under heating, forming by filling followed by solidification under cooling, or comprises subjecting the components to forming by filling or by pressing in a wet condition followed by drying.

A research has been also made on a process for producing intraorallydisintegrable pharmaceutical preparations by means of a wet forming technique, which has been most widely employed in production of tablets. JP-A-9-48726 discloses an intraorally rapidly disintegrable preparation which is obtained by wetting drug-containing fine granules by humidification, compression molding and then by incorporating a material to retain the shape of the formulation. As the material, sugars, sugar alcohols, and water-soluble polymer materials are exemplified. JP-A-5-271054 discloses a process comprising tableting and drying a mixture of a drug, sugar, and water as to moisten the sugar particle surface. JP-A-8-291051 discloses a process comprising tableting a mixture of a drug, water-soluble binder and water-soluble excipient under low pressure followed by humidification and drying.

In the above processes, however, it is difficult to use a conventional tableting machine because a moistened mixture is used in tableting; there is another problem that multiple steps are required in the production because humidification and drying are required after tableting; in addition, the processes have another serious problem that they cannot be applied to drugs unstable in water.

JP-T-6-502194 (USP 5,464,632) discloses rapidly disintegrable multi-granular tablets which comprise an effective ingredient in a form of coated microcrystals or in a form of coated or uncoated fine particles and a mixture of excipients and which disintegrate within a very short time of 60 seconds in the mouth. The reference also describes addition of a disintegrator to the tablets, however, the disintegrator is not specifically defined therein. Further, there is no description in the reference as to the addition of D-mannitol as excipient.

JP-A-11-35450 discloses improved multi-particulate tablets which comprise an effective ingredient in a form of coated microcrystals and excipients and decompose within 40 seconds in the mouth. The tablets contain as excipients at least one decomposing agent such as crospovidone and a polyol such as mannitol, wherein the excipient are in a formof a directly compressible molding having an average particle size of 100 to 500 µm or in a form of powder having an average particle size of less than 100 µm.

On the other hand, D-mannitol is excellent in safety, well mixed with physiologically active substances, has no hygroscopicity, and hardly retain water. Accordingly, D-mannitol is an excipient valuable particularly for use in preparation of tablets or capsules containing a physiologically active substance, which is sensitive to water. However, D-mannitol is poor in binding ability during compression molding with great friction with a metal wall, and accordingly, the use of D-mannitol as an excipient might causes die friction or capping during compression molding and fails to give sufficient hardness to the tablets. Further, D-mannitol causes abrasion in the wall surface of a die and in the side surface of a punch in a tablet machine (punching machine), and sometimes makes the operation of the machine difficult. In the circumstance, the use of D-mannitol is very limited to the particular formulation such as chewable tablets.

Meanwhile, D-mannitol is known to be crystalline powder having crystal polymorphism classified into the α-type, β-type and δ-type based on the X-diffraction pattern [Walter-Levy, L., Acad. Sci. Parist. 276 Series C, 1779 (1968)]. As to the improvement in moldability of crystalline powder, i.e., the improvement in binding ability during compression molding, it is known in general that pulverizaion of crystals increases the number of the binding point (contact area between particles) of crystals, which results in improvement in moldability (binding ability during compression molding). However, pulverization of D-mannitol not only promotes friction with the surface of metal wall during compression molding but also causes a problem in handling such as scattering or decrease of fluidity.

Therefore, in the prior art, D-mannitol alone is rarely used as an excipient in compression moldings, but it is used in combination with other well-molding excipients such as sugars and with other additives such as binders or fillers.

As for a process for improving the moldability of D-mannitol, JP-A-61-85330 discloses a process for producing excipients for direct tabletting which comprises spray-drying D-mannitol; and JP-A-10-36291 discloses a solid composition which contains D-mannitol having a specific surface area of about 1 m²/g as an excipient excellent in compression molding. In these processes, however, the processing of D-mannitol is complicated, and expensive. In production of tablets, it is feared that the uniformity of the content of an active ingredient is decreased due to variation of the tablet weight caused by insufficient fluidity of the fine granules and that the uniformity of an active ingredient contained in the tablets is decreased due to insufficient mixing with the active ingredient.

WO97/47287 discloses tablets which contain sugar alcohols including D-mannitol having an average particle size of 30 µm or less.

In particular, when a water-soluble drug is used as a pharmacologically active ingredient, it is desired to further reduce the time required for disintegration of the preparation in the oral cavity.

### Disclosure of the Invention

An object of the present invention is to provide an intraorally rapidly disintegrable tablet which rapidly disintegrates in the oral cavity without water and have the practical hardness so as not to cause trouble such as losing its shape during the production process and distribution or in the course of handling in hospitals or by patients, and a process for producing the tablet thereof.

The present inventors conducted various investigation, and found that an intraorally rapidly disintegrable tablet having excellent disintegrability and practical hardness can be obtained addition of D-mannitol and a disintegrator to fine granules prepared by granulating a mixture of a water-soluble pharmacologically active ingredient and an adsorbent.

In addition, the present inventors also conducted various investigation as to a process for producing such intraorally rapidly disintegrable tablets and found that an intraorally rapidly disintegrable tablet which have practically trouble-free hardness and rapidly disintegrates in an oral cavity can be obtained by mixing D-mannitol and a disintegrator with fine granules prepared by granulating a mixture of a water-soluble pharmacologically active ingredient and an adsorbent and then by subjecting the resulting compression molding material to compression molding.

In particular, the inventors found that the time required for disintegration of the tablets can further be reduced by means of compression molding of a compression molding material using a compression molding machine in which a lubricant is previously applied on the surface of punch and the die wall (referred to as an external-lubricating tableting method).

That is, the present invention relates to the following items (1) to (76).
(1) An intraorally rapidly disintegrable tablet which comprises fine granules prepared by granulating a mixture of a water-soluble pharmacologically active ingredient and an adsorbent, D-mannitol and a disintegrator.
(2) The intraorally rapidly disintegrable tablet as described in the above item (1), wherein the adsorbent is at least one member selected from the group consisting of calcium silicate, light anhydrous silicic acid, synthetic aluminum silicate, silicon dioxide and magnesium metasilicate aluminate.
(3) The intraorally rapidly disintegrable tablet as described in the above item (1) or (2), wherein the disintegrator is at least one member selected from the group consisting of crospovidone, low-substituted hydroxypropylcellulose, croscarmellose sodium and carboxymethylcellulose.
(4) The intraorally rapidly disintegrable tablet as described in the above items (1) to (3), wherein the whole or a part of the D-mannitol is a primary particle and the specific surface area of the primary particle is 1.0 m²/g or less.
(5) The intraorally rapidly disintegrable tablet as described in the above items (1) to (4), wherein the solubility of the water-soluble pharmacologically active ingredient in water is 1 mg/ml or more.
(6) The intraorally rapidly disintegrable tablet as described in the above items (1) to (4), wherein the water-soluble pharmacologically active ingredient is pravastatin sodium.
(7) The intraorally rapidly disintegrable tablet as described in the above items (1) to (6), further containing a lubricant.
(8) The intraorally rapidly disintegrable tablet as described in the above item (7), wherein the lubricant is contained only on the surface of the tablet.
(9) The intraorally rapidly disintegrable tablet as described in the above item (7) or (8), wherein the lubricant is at least one member selected from the group consisting of magnesium stearate, calcium stearate, stearic acid, stearyl alcohol, sodium stearyl fumarate, sucrose fatty acid ester and talc.
(10) The intraorally rapidly disintegrable tablet as described in the above items (1) to (9), further containing at least one member selected from the group consisting of flavoring agents, sweeteners, perfumes, coloring agents, stabilizers, fluidizing agents, anti-oxidants and co-solubilizers.
(11) The intraorally rapidly disintegrable tablet as described in the above items (1) to (10), wherein the compounding ratio of the water-soluble pharmacologically active ingredient to the adsorbent in the fine granules is 1:10 to 10:1.
(12) The intraorally rapidly disintegrable tablet as described in the above items (1) to (11), wherein the compounding ratio of the fine granules in the tablet is 1 to 50% by weight.
(13) The intraorally rapidly disintegrable tablet as described in the above items (1) to (12), wherein the compounding ratio of the D-mannitol in the tablet is 20 to 99% by weight.
(14) The intraorally rapidly disintegrable tablet as described in the above items (1) to (13), wherein the compounding ratio of the disintegrator in the tablet is 0.5 to 30% by weight.
(15) The intraorally rapidly disintegrable tablet as described in the above items (1) to (14), wherein the hardness of the tablet is 20N or higher.
(16) The intraorally rapidly disintegrable tablet as described in the above items (1) to (15), wherein the disintegration time in oral cavity is 30 seconds or less.
(17) A process for producing an intraorally rapidly disintegrable tablet which comprises mixing fine granules prepared by granulating a mixture of a water-soluble pharmacologically active ingredient and an adsorbent, D-mannitol and a disintegrator to prepare a material for compression molding, and subjecting the material to compression molding.
(18) The process as described in the above item (17), wherein the adsorbent is at least one member selected from the group consisting of calcium silicate, light anhydrous silicic acid, synthetic aluminum silicate, silicon dioxide and magnesium metasilicate aluminate.
(19) The process as described in the above item (17) or (18), wherein the disintegrator is at least one member selected from the group consisting of crospovidone, low-substituted hydroxypropylcellulose, croscarmellose sodium and carboxymethylcellulose.
(20) The process as described in the above items (17) to (19), wherein the whole or a part of the D-mannitol is a primary particle and the specific surface area of the primary particle is 1.0 m²/g or less.
(21) The process as described in the above items (17) to (20), wherein the solubility of the water-soluble pharmacologically active ingredient in water is 1 mg/ml or more.
(22) The process as described in the above items (17) to (20), wherein the water-soluble pharmacologically active ingredient is pravastatin sodium.
(23) The process as described in the above items (17) to (22), wherein the material for compression molding contains a lubricant.
(24) The process as described in the above items (17) to (23), wherein the compression molding is carried out using a compression molding machine in which a lubricant is previously applied on the surface of punch and the die.
(25) The process as described in the above item (23) or (24), wherein the lubricant is at least one member selected from the group consisting of magnesium stearate, calcium stearate, stearic acid, stearyl alcohol, sodium stearyl fumarate, sucrose fatty acid ester and talc.
(26) The process as described in the above items (17) to (25), wherein the material for compression molding contains at least one member selected from the group consisting of flavoring agents, sweeteners, perfumes, coloring agents, stabilizers, fluidizing agents, anti-oxidants and co-solubilizers.
(27) The process as described in the above items (17) to (26), wherein the compounding ratio of the water-soluble pharmacologically active ingredient to the adsorbent in the fine granules is 1:10 to 10:1.
(28) The process as described in the above items (17) to (27), wherein the compounding ratio of the fine granules in the tablet is 1 to 50% by weight.
(29) The process as described in the above items (17) to (28), wherein the compounding ratio of the D-mannitol in the tablet is 20 to 99% by weight.
(30) The process as described in the above items (17) to (29), wherein the compounding ratio of the disintegrator in the tablet is 0.5 to 30% by weight.
(31) An intraorally rapidly disintegrable tablet which is produced by mixing fine granules prepared by granulating a mixture of a water-soluble pharmacologically active ingredient and an adsorbent D-mannitol and a disintegrator to prepare a material for compression molding and subjecting the material to compression molding.
(32) The intraorally rapidly disintegrable tablet as described in the above item (31), wherein the adsorbent is at least one member selected from the group consisting of calcium silicate, light anhydrous silicic acid, synthetic aluminum silicate, silicon dioxide and magnesium metasilicate aluminate.
(33) The intraorally rapidly disintegrable tablet as described in the above item (31) or (32), wherein the disintegrator is at least one member selected from the group consisting of crospovidone, low-substituted hydroxypropylcellulose, croscarmellose sodium and carboxymethylcellulose.
(34) The intraorally rapidly disintegrable tablet as described in the above items (31) to (33), wherein whole or a part of the D-mannitol is a primary particles and the specific surface area of the primary particle is 1.0 m²/g or less.
(35) The intraorally rapidly disintegrable tablet as described in the above items (31) to (34), wherein the solubility of the water-soluble pharmacologically active ingredient in water is 1 mg/ml or more.
(36) The intraorally rapidly disintegrable tablet as described in the above items (31) to (34), wherein the water-soluble pharmacologically active ingredient is pravastatin sodium.
(37) The intraorally rapidly disintegrable tablet as described in the above items (31) to (36),wherein the material for compression molding contains a lubricant.
(38) The intraorally rapidly disintegrable tablet as described in the above items (31) to (37), wherein the compression molding is carried out using a compression molding machine in which a lubricant is previously applied on the surface of punch and the die.
(39) The intraorally rapidly disintegrable tablet as described in the above item (37) or (38), wherein the lubricant is at least one member selected from the group consisting of magnesium stearate, calcium stearate, stearic acid, stearyl alcohol, sodium stearyl fumarate, sucrose fatty acid ester and talc.
(40) The intraorally rapidly disintegrable tablet as described in the above items (31) to (39), wherein the material for compression molding contains at least one member selected from the group consisting of flavoring agents, sweeteners, perfumes, coloring agents, stabilizers, fluidizing agents, anti-oxidants and co-solubilizers.
(41) The intraorally rapidly disintegrable tablet as described in the above items (31) to (40), wherein the compounding ratio of the water-soluble pharmacologically active ingredient to the adsorbent in the fine granules is 1:10 to 10:1.
(42) The intraorally rapidly disintegrable tablet as described in the above items (31) to (41), wherein the compounding ratio of the fine granules in the tablet is 1 to 50% by weight.
(43) The intraorally rapidly disintegrable tablet as described in the above items (31) to (42), wherein the compounding ratio of the D-mannitol in the tablet is 20 to 99% by weight.
(44) The intraorally rapidly disintegrable tablet as described in the above items (31) to (43), wherein the compounding ratio of the disintegrator in the tablet is 0.5 to 30% by weight.
(45) The intraorally rapidly disintegrable tablet as described in the above items (31) to (44), wherein the hardness of the tablet is 20N or higher.
(46) The intraorally rapidly disintegrable tablet as described in the above items (31) to (45), wherein the disintegration time in oral cavity is 30 seconds or less.
(47) A process for producing an intraorally rapidly disintegrable tablet which comprises granulating a mixture of a water-soluble pharmacologically active ingredient, an adsorbent, D-mannitol and a disintegrator to prepare a material for compression molding, and subjecting the material to compression molding.
(48) The process as described in the above item (47), wherein the adsorbent is at least one member selected from the group consisting of calcium silicate, light anhydrous silicic acid, synthetic aluminum silicate, silicon dioxide and magnesium metasilicate aluminate.
(49) The process as described in the above item (47) or (48), wherein the disintegrator is at least one member selected from the group consisting of crospovidone, low-substituted hydroxypropylcellulose, croscarmellose sodium and carboxymethylcellulose.
(50) The process as described in the above items (47) to (49), wherein whole or a part of the D-mannitol is a primary particle and the specific surface area of the primary particle is 1.0 m²/g or less.
(51) The process as described in the above items (47) to (50), wherein the solubility of the water-soluble pharmacologically active ingredient in water is 1 mg/ml or more.
(52) The process as described in the above items (47) to (50), wherein the water-soluble pharmacologically active ingredient is pravastatin sodium.
(53) The process as described in the above items (47) to (52), wherein the material for compression molding contains a lubricant.
(54) The process as described in the above items (47) to (53), wherein the compression molding is carried out using a compression molding machine in which a lubricant is previously applied on the surface of punch and the die.
(55) The process as described in the above item (53) or (54), wherein the lubricant is at least one member selected from the group consisting of magnesium stearate, calcium stearate, stearic acid, stearyl alcohol, sodium stearyl fumarate, sucrose fatty acid ester and talc.
(56) The process as described in the above items (47) to (55), wherein the compression molding material containis at least one member selected from the group consisting of flavoring agents, sweeteners, perfumes, coloring agents, stabilizers, fluidizing agents, anti-oxidants and co-solubilizers.
(57) The process as described in the above items (47) to (56), wherein the compounding ratio of the water-soluble pharmacologically active ingredient to the adsorbent in the compression molding material is 1:10 to 10:1.
(58) The process as described in the above items (47) to (57), wherein the compounding ratio of the water-soluble pharmacologically active ingredient and the adsorbent in the tablet is 1 to 50% by weight.
(59) The process as described in the above items (47) to (58), wherein the compounding ratio of the D-mannitol in the tablet is 20 to 99% by weight.
(60) The process as described in the above items (47) to (59), wherein the compounding ratio of the disintegrator in the tablet is 0.5 to 30% by weight.
(61) An intraorally rapidly disintegrable tablet which comprises a water-soluble pharmacologically active ingredient, an adsorbent, D-mannitol and a disintegrator.
(62) The intraorally rapidly disintegrable tablet as described in the above item (61), wherein the adsorbent is at least one member selected from the group consisting of calcium silicate, light anhydrous silicic acid, synthetic aluminum silicate, silicon dioxide and magnesium metasilicate aluminate.
(63) The intraorally rapidly disintegrable tablet as described in the above item (61) or (62), wherein the disintegrator is at least one member selected from the group consisting of crospovidone, low-substituted hydroxypropylcellulose, croscarmellose sodium and carboxymethylcellulose.
(64) The intraorally rapidly disintegrable tablet as described in the above items (61) to (63), wherein whole or a part of the D-mannitol is a primary particle and the specific surface area of the primary particle is 1.0 m²/g or less.
(65) The intraorally rapidly disintegrable tablet as described in the above items (61) to (64), wherein the solubility of the water-soluble pharmacologically active ingredient in water is 1 mg/ml or more.
(66) The intraorally rapidly disintegrable tablet as described in the above items (61) to (64), wherein the water-soluble pharmacologically active ingredient is pravastatin sodium.
(67) The intraorally rapidly disintegrable tablet as described in the above items (61) to (66), containing a lubricant.
(68) The intraorally rapidly disintegrable tablet as described in the above item (67), wherein the lubricant is contained only on the surface of the tablet.
(69) The intraorally rapidly disintegrable tablet as described in the above item (67) or (68), wherein the lubricant is at least one member selected from the group consisting of magnesium stearate, calcium stearate, stearic acid, stearyl alcohol, sodium stearyl fumarate, sucrose fatty acid ester and talc.
(70) The intraorally rapidly disintegrable tablet as described in the above items (61) to (69), further containing at least one member selected from the group consisting of flavoring agents, sweeteners, perfumes, coloring agents, stabilizers, fluidizing agents, anti-oxidants and co-solubilizers.
(71) The intraorally rapidly disintegrable tablet as described in the above items (61) to (70), wherein the compounding ratio of the water-soluble pharmacologically active ingredient to the adsorbent is 1:10 to 10:1.
(72) The intraorally rapidly disintegrable tablet as described in the above items (61) to (71), wherein the compounding ratio of the water-soluble pharmacologically active ingredient and the adsorbent in the tablet is 1 to 50% by weight.
(73) The intraorally rapidly disintegrable tablet as described in the above items (61) to (72), wherein the compounding ratio of the D-mannitol in the tablet is 20 to 99% by weight.
(74) The intraorally rapidly disintegrable tablet as described in the above items (61) to (73), wherein the compounding ratio of the disintegrator in the tablet is 0.5 to 30% by weight.
(75) The intraorally rapidly disintegrable tablet as described in the above items (61) to (74), wherein the hardness of the tablet is 20N or higher.
(76) The intraorally rapidly disintegrable tablet as described in the above items (61) to (75), wherein the disintegration time in the oral cavity is 30 seconds or less.

Any water-soluble pharmacologically active ingredient may be used in the present invention, as far as it is a pharmacologically active ingredient soluble in water. However, it is preferred that the active ingredient has, for example, the solubility of 1 mg/ml or more in water, more preferably 10 mg/ml or more, and even more preferably 100 mg/ml.

The solubilityof the pharmacologically active ingredient can be determined according to the Japanese Pharmacopoeia 13, General Notices 23, specifically by adding powder into water, vigorously shaking the mixture at 20±5°C for 30 seconds every 5 minutes, and measuring the degree of dissolution within 30 minutes.

The intraorally rapidly disintegrable tablet includes one or two or more of pharmacologically active ingredients selected from the group consisting of antipyretics, analgesics, anti-inflammatory agents, antipyretic analgesic anti-inflammatory agents, psychotropic agents, psychopharmaceuticals, anti-anxiety agents, anti-depressants, CNS agents, medicines for the digestive organs, anti-ulcer agents, agents for peptic ulcers such as gastric antacids, hypnotic sedatives, drugs for improving brain metabolism, anti-tussives, expectrants, anti-emetics, drugs for motion sickness, anti-allergic agents, bronchodilators, drugs for bronchial asthma, cardiotonics, anti-arrhythmic agents, anti-histaminic agents, diuretics, hypotensives, vasoconstrictors, cholagogues, antihypertensives, drugs for hyperlipemia, anti-diabetic agents, antiepilaptics, drugs for Parkinson's disease, vasodilators such as coronary vasodilators and peripheral vasodilators, drugs for cerebrovascular accidents, antibiotics, drugs for Alzheimer's disease, and drugs for gout.

Examples of the pharmacologically active ingredient having the solubility in water of 100 mg/ml or more include:
drugs for hyperlipemia such as pravastatin sodium and cerivastatin sodium;
antipyretic analgesic anti-inflammatory agents such as loxoprofen sodium;
hypnotic sedatives such as flurazepam hydrochloride;
antiepilaptics such as sodium valproate;
drugs for Parkinson's disease such as amantadine hydrochloride;
psychotropic agents such as imipramine hydrochloride;
cardiotonics such as etilefrine hydrochloride;
anti-arrhythmic agents such as oxprenolol hydrochloride and mexiletine hydrochloride;
vasodilators such as diltiazem;
anti-tussives such as pentoxyverine citrate;
expectrants such as L-methylcysteine hydrochloride;
drugs for peptic ulcers such as pirenzepine hydrochloride; and the like.

The water-soluble pharmacologically active ingredient contained in the intraorally rapidly disintegrable tablets of the present invention may be in a form of pharmacologically acceptable salts as far as they are soluble in water. The pharmacologically acceptable salts include, for example, salts with inorganic acids (e.g., hydrochloric acid, sulfuric acid, nitric acid, etc.), organic acids (e.g., carbonic acid, bicarbonic acid, succinic acid, acetic acid, propionic acid, trifluoroacetic acid, etc.), inorganic bases (e.g., alkali metal such as sodium, potassium, etc.; alkaline earth metal such as calcium, magnesium, etc.), and organic bases (e.g., organic amines such as triethylamine; basic amino acids such as arginine).

Examples of the adsorbent contained in the intraorally rapidly disintegrable tablets of the present invention includes, silicic acids and their derivatives such as calcium silicate, light anhydrous silicic acid, synthetic aluminum silicate, silicon dioxide, and magnesium metasilicate aluminate. Particularly preferred is calcium silicate.

As D-mannitol contained in the intraorally rapidly disintegrable tablets of the present invention, commercially available one may usually be used, which may be further processed by spray drying. It is preferable that the whole or a part of D-mannitol exists in a state of primary particles, which may preferably be prepared in a form of primary particles having the desired average particle size and specific surface area by a pulverizer or a sieving machine. The average particle size of the primary particles is preferably in the range of 10 to 300 µm, more preferably, 30 to 100 µm. The specific surface area of the primary particles of D-mannitol is preferably in the range of 1.0 m²/g or less, more preferably 1.0 to 2.1×10⁻⁶ m²/g, even more preferably 0.4 to 0.02 m²/g.

In this context, the average particle size means 50% particle size, more specifically the particle size at 50% in the cumulative percentage graph. The particle size may be measured, for example, by a laser diffraction method for measuring particle size distribution, more specifically, a method using a laser diffraction/scattering particle size distribution-measuring apparatus LA-910 (Horiba Seisakusyo). The specific surface area may be measured according to the BET (Brunauer-Emmett and Teller's) way, for example, using a Horiba-Seisakusyo's apparatus type SA-9603.

Examples of disintegrator contained in the intraorally rapidly disintegrable tablets of the present invention include crospovidone, low-substituted hydroxypropylcellulose, croscarmellose sodium, carboxymethylcellulose, and the like, and crospovidone is preferred. Crospovidone may be in any form as far as it is a crosslinking polymer of 1-vinyl-2-pyrrolidone; usually, crospovidone having the molecular weight of 1,000,000 or more may be used. The crospovidone may be prepared in a known process; alternatively, commercially available Kollidon CL (BASF Japan) or Polyplasdon XL (ISP Japan) may be employed as crospovidone.

In the intraorally rapidly disintegrable tablets of the present invention, there is no limitation for the ratio of the water-soluble pharmaceutically active ingredient to the adsorbent (the weight of the water-soluble pharmaceutically active ingredient : the weight of the adsorbent) contained in the fine granules prepared by granulating a mixture of a water-soluble pharmacologically active ingredient and an adsorbent is not particularly limited, and the preferred ratio is, for example, 1:10 to 10:1, more preferably 1: 5 to 5:1, even more preferably 1:2 to 2:1.

In the intraorally rapidly disintegrable tablets of the present invention, there is no limitation for the ratio of the fine granules prepared by granulating a mixture of a water-soluble pharmacologically active ingredient and an adsorbent in the tablet (the ratio of the fine granules by weight prepared by granulating a mixture of a water-soluble pharmacologically active ingredient and an adsorbent to the total weight of the tablet); and the preferred ratio is, for example, 1 to 50% by weight, more preferably 1 to 40% by weight, and even more preferably 1 to 30% by weight.

In the intraorally rapidly disintegrable tablets of the present invention, there is no limitation for the ratio of D-mannitol (the ratio of D-mannitol by weight to the total weight of the tablet); and the preferred rate is, for example, 20 to 99% by weight, more preferably 40 to 99% by weight, and even more preferably 60 to 99% by weight.

In the intraorally rapidly disintegrable tablets of the present invention, there is no limitation for the ratio of the disintegrator (the ratio of disintegrator by weight to the total weight of the tablet); and the preferred rate is 0.5 to 30% by weight, more preferably 0.5 to 20% by weight, and even more preferably 1 to 10% by weight.

In the rapidly disintegrable tablets of the invention, the ratio of the fine granules prepared by granulating a mixture of a water-soluble pharmacologically active ingredient and an adsorbent, D-mannitol and the disintegrator, may properly be determined depending on the solubility of the water-soluble pharmacologically active ingredient, particle size, physical properties such as wetting property to water, kinds of the adsorbent and disintegrator, and so on. It is preferred to add a lubricant in the intraorally rapidly disintegrable tablets of the present invention. The lubricant is not particularly limited, and the examples includes magnesium stearate, calcium stearate, stearic acid, stearyl alcohol, sodium stearyl fumarate, sucrose fatty acid ester, talc, and the like.

The ratio of the lubricant to be added to the intraorally rapidly disintegrable tablets of the present invention is preferably 0.001 to 2% by weight, more preferably 0.01 to 1% by weight, and even more preferably 0.05 to 0.5% by weight.

Though the lubricant may be contained in the inner part of the intraorally rapidly disintegrable tablets of the present invention, it is more preferable that the lubricant is retained only on the surface of the tablets by an external-lubricating tableting method as described below.

The intraorally rapidly disintegrable tablets of the present invention, if required, may contain one or more additive components selected from the group consisting of corrigants, sweeteners, flavors, coloring agents, stabilizers, fluidizing agents, anti-oxidants and auxiliary solubilizers. The ratio of the additive to be added to the total weight of tablet is preferably 5% or less by weight, more preferably 1% or less by weight, and even more preferably 0.5% by weight.

Examples of the corrigants or sweeteners include aspartame, saccharin sodium, stevia, glycyrrhizin dipotassium, and the like.

Examples of the flavor include L-menthol, lemon, orange, strawberry, mint, and the like.

Examples of the coloring agents include yellow iron sesquioxide, red iron sesquioxide, food pigments such as Food Yellow No.5, Food Blue No.2, food lake pigments, and the like.

Examples of the stabilizers include benzoic acid, sodium benzoate, citric acid, and the like.

Examples of the fluidizing agents include light anhydrous silicic acid, calcium silicate, synthetic aluminum silicate, and the like.

Examples of the anti-oxidants include ascorbic acid, tocopherol, and the like.

Examples of the auxiliary solubilizers includesurfactants such as polysorbate 80, sodium laurylsulfate, macrogol 400, and the like.There is no limitation for the additives to be added to the intraorally rapidly disintegrable tablets of the present invention, and preferably the tablets do not contain binders or sugars other than D-mannitol, which show high viscosity on contact with water. In the absence of any sugar other than D-mannitol and a binder, the resulting intraorally rapidly disintegrable tablets show a tendency to improve their disintegration in the oral cavity.

Examples of the sugars other than D-mannitol include sugars such as white soft sugar, glucose, maltose, fructose; sugar alcohols such as sorbitol, maltitol, and the like. Examples of the binders include hydroxypropylcellullose (HPC), hydroxypropylmethylcellulose (HPMC), methylcellulose (MC), polyvinylpyrrolidone (PVP), pullulan, polyvinyl alcohol (PVA), gelatin, and the like.

The intraorally rapidly disintegrable tablets of the present invention have practical hardness of tablets and rapidly disintegrate without water in the oral cavity.

In this context, the term "practical hardness of tablets" means that the tablets have the hardness of 20N or more, preferably 30N or more, although the hardness vary depending on the component and shape of the tablets. The hardness of tablets can easily be measured by means of a known tablet-hardness indicator, for example, Toyama Type TH-303 (Toyama Chemical Co., Ltd.).

The term "rapid disintegration" means that the tablets disintegrate with saliva in the oral cavity without water within a practically appropriate time. "The practically appropriate time for disintegration" is 30 seconds or shorter, though the time varies depending on a recipient. The disintegration time can be obtained by measuring the time required for the complete disintegration of the tablets with the saliva in the mouth of a healthy adult.

The intraorally rapidly disintegrable tablets of the present invention are not limited to the administration without water, and a patient whose secretion of the saliva is poor can also take the tablets with water, and thus the tablets of the present invention can be taken much more easily than the usual ones. That is, the intraorally rapidly disintegrable tablets of the present invention may be taken, for example, according to the following manners: (a) the tablet is kept in the mouth without swallowing and dissolved or integrated with saliva in the oral cavity with a small quantity of water or without water; (b) the tablet is swallowed directly with water; and (c) the tablet is dissolved or disintegrated in water and then taken.

The intraorally rapidly disintegrable tablets of the present invention can be taken without water as mentioned above, and accordingly are particularly preferable in the following cases: (i) in the case in which administration is required frequently without water; (ii) in the case that the recipient is a patient who is difficult to swallow the tablet; and (iii) in the case that the recipient is an aged person or infant who tends to choke over the tablets of an old type. Examples of drugs in the cases (i) to (iii) are therapeutics (sometimes used as prophylactics depending on diseases) such as antipyretics, analgesics, anti-inflammatory agents, anti-anxiety agents, anti-tussives, expectrants, anti-emetics, drugs for motion sickness, antihypertensives, drugs for hyperlipemia, anti-diabetic agents, drugs for bronchial asthma, drugs for cerebrovascular accidents, and the like.

The intraorally rapidly disintegrable tablets of the present invention can be produced by a conventional method employed in a field of pharmaceutical preparations such as a direct tableting or indirect tableting method. For example, a mixture of a water-soluble pharmacologically active ingredient and an adsorbent is granulated, and the resulting fine granules are mixed with D-mannitol, a disintegrator and optionally other additives such as lubricants, and molded under compression.

The "mixing" may be carried out using an apparatus such as Vertical Granulator VG10 (Powrex Corporation), universal kneader, fluid bed granulator, V-type mixer, tumbler mixer, and so on. The "compression molding" may be carried out using a known tablet machine, for example, single tablet machine, rotary-type tablet machine, and so on.

The fine granules, which are used in the rapidly disintegrating buccal tablets of the invention and prepared from a mixture of a water-soluble pharmacologically active ingredient and an adsorbent, may be prepared in any way with no particular limitation, for example, a wet granulation method as follows; 1) a water-soluble pharmacologically active ingredient is mixed with an adsorbent, granulated with addition of an aqueous medium, and dried; and 2) a water-soluble pharmacologically active ingredient dissolved or dispersed in an aqueous medium is added to an adsorbent, granulated, and dried. In this situation, other additive may be added thereto in addition to a water-soluble pharmacologically active ingredient and an adsorbent. Examples of the other additives include a part of D-mannitol and disintegrator to be added to the intraorally rapidly disintegrable tablets of the present invention, the lubricants, corrigants, sweeteners, flavors, fluidizing agents, stabilizers, anti-oxidants and auxiliary solubilizers.

Examples of the aqueous medium include purified water, methanol, ethanol, acetone, and amixture thereof, and the mixing ratio may be suitably determined. Among them, purified water is preferred. The amount of the aqueous medium for granulation to be added to the above fine granules is preferably 5 to 30% by weight.

There is no particular limitation for the process for the production of the intraorally rapidly disintegrable tablets of the present invention, which contain fine granules, D-mannitol and a disintegrator, said fine granules being prepared by granulating a mixture of the above-mentioned water-soluble pharmacologically active ingredient and adsorbent, and mentioned is a method comprising a step for preparing a material for compression molding from the whole components or a part thereof by wet granulation, e.g., fluid bed granulation, oscillating fluid bed granulation, stirring granulation (preferably, high-speed stirring granulator NSK-250 (Okada Seiko) or Porex high-speed stirring granulator VG-25 is used), or extrusion granulation (preferably, Fuji Paudal DGL-1 is used).

Specific examples of the method include: a method in which a mixture of a water-soluble pharmacologically active ingredient and an adsorbent is granulated, and the resulting fine granules are mixed with D-mannitol and a disintegrator, again granulated with an aqueous medium in the same manner as above, dried to yield fine granules, and other additives are added thereto, followed by compression molding; a method in which fine granules containing D-mannitol and a disintegrator are mixed with the above-mentioned fine granules prepared by granulating a mixture of a water-soluble pharmacologically active ingredient and an adsorbent, followed by compression molding; and a method which comprises the following steps:
(1) a step in which a mixture of a water-soluble pharmacologically active ingredient and an adsorbent is granulated to produce fine granules (a granulation product containing a water-soluble pharmacologically active ingredient and an adsorbent);
(2) a step in which D-mannitol is granulated to yield fine granules (a granulation product of D-mannitol);
(3) a step in which the granulation product containing a water-soluble pharmacologically active ingredient and an adsorbent is mixed with the granulation product of D-mannitol and a disintegrator to yield a material for compression molding; and
(4) a step in which the material for compression molding is subjected to compression molding.

In addition, as aprocess forproductionof the intraorally rapidly disintegrable tablets which comprises mixing and granulating a water-soluble pharmacologically active ingredient, an adsorbent, D-mannitol and a disintegrator, mentioned is a method which comprises:
(1) a step in which a mixture of a water-soluble pharmacologically active ingredient, an adsorbent, D-mannitol and a disintegrator is granulated to yield a compression molding material; and
(2) a step in which the compression molding material is subjected to compression molding.

The particle size and specific surface area of the granulation product are not limited and suitably adjusted as far as the fluidity is kept with giving no trouble to the tableting.

When a lubricant is added to the intraorally rapidly disintegrable tablets of the present invention, for example, the lubricant may be added and mixed with fine granules prepared by granulating a mixture of a water-soluble pharmacologically active ingredient and an adsorbent and fine granules comprising D-mannitol and a disintegrator. The above-mentioned other additives other than the lubricant (for example, flavors, stabilizers, etc. ) maybe added and mixed with the fine granules.

The granulation product may be dried in any way without particular limitation using a known dryer, for example, draft box-type drier (preferably, Japan Dryer TE-98) at 40 to 80°C over a period of about 30 to 90 minutes, or using a fluid bed dryer (preferably, Freund Sangyo FLO-5) at 70 to 90°C over a period of about 5 to 30 minutes.

Alternatively, using a fluid bed granulation dryer (preferably, Freund Sangyo FLO-2), the granulation and the drying of the resulting granulation product can be carried out at once.

Subsequently, if required, an additive other than the lubricant, such as flavors or stabilizers, may be added to the resulting granulation product to yield a material for compression molding. Then, the material for compression molding is subjected to compression molding to yield the intraorally rapidly disintegrable tablets of the present invention. The compression molding may be carried out using a known tablet machine, preferably, a single tablet machine, rotary-type tablet machine, oil press, and so on. The pressure in the compression molding is 3 to 30 kN, preferably 5 to 20 kN.

Another embodiment of the process for the production of the intraorally rapidly disintegrable tablets of the present invention is a method in which fine granules prepared by granulating a mixture of a water-soluble pharmacologically active ingredient and an adsorbent, D-mannitol, a disintegrator, and if required a lubricant and the other additive mentioned above are mixed using a known mixer, for example, V-type mixer, tumbler mixer, and so on, and the resulting mixture is subjected to direct tableting.

In the process for the producing the intraorally rapidly disintegrable tablets of the present invention, although a lubricant may be contained in the inner part of the tablet together with other additives as mentioned above, it is preferred that a lubricant without mixing with the other components may be applied previously on the surface of the punch and the die wall and then the material for compression molding is molded under compression (external-lubricating tableting method),which is advantageous in increasing the hardness and shortening the disintegration time of the tablets. There is no particular limitation for a method for applying the lubricant to the punch and the die.

There is no particular limitation for the weight and shape of the intraorally rapidly disintegrable tablets of the present invention. For example, the pharmaceutical preparation may be in a circular shape or in various modified shapes having such a surf ace shape as normal R-surf ace, sugar-coated R-surface, corner square surface, corner round surface, double R-surface, etc. The tablet may be in a dividable form having a cleavage line. The tablet may also be multi-layered one having two or more layers.

It is particularly preferred that the intraorally rapidly disintegrable tablets of the present invention are preferably in a small size. The hardness and the time required for disintegration can be controlled by the weight and/or shape of the tablet. The intraorally rapidly disintegrable tablets of the present invention, preferably have a weight of 80 to 250 mg and a tablet size of 6 to 9 mm.

The intraorally rapidly disintegrable tablets of the present invention can be administered orally and safely not only to human but also to other mammals (e.g., mouse, rat, rabbit, cat, dog, bovine, horse, monkey, human, etc.). The dose of the intraorally rapidly disintegrable tablets of the present invention may be suitably determined in the range that the water-soluble pharmacologically active ingredient works effectively, though it may vary depending on the species of the water-soluble pharmacologically active ingredient, the recipient, and disease. The intraorally rapidly disintegrable tablets of the present invention may be administered once or multiple times a day, preferably divided into 2 to 4 parts a day.

### Best Mode for Carrying Out the Invention

The present invention will be explained in more details by referring to the following Examples and Test Example, which are not intended to limit the present invention.

In this context, PVS means pravastatin sodium.

### Comparative Example 1

In a high-speed stirring granulator (Powrex; Type VG-25) was placed 200 g of PVS, 1992 g of D-mannitol (Nikken Chemical & Synthetic Ind.; average particle size 50 µm, specific surface area 0.17 m²/g) and 148 g of crospovidone, and the mixture was agitated for 5 minutes. Purified water was added thereto, which was subjected to granulation. The granules were dried on a fluid bed at a suction temperature of 80°C and sieved through a No. 20 wire gauze. To the sieved granules (1170 g) was added 18 g of aspartame, 12 g of magnesium stearate and 1 g of mint flavor to give granules for tableting. Using a rotary-type tablet machine (HATA-AP15; Hata Iron Works) equipped with a punch of 7 mmφ flat type, the granules were subjected to compression molding under pressure of 700 kg to yield tablets of 120 mg/tablet.

### Example 1

In a high-speed stirring granulator was placed 500 g of PVS and 240 g of calcium silicate ("Flow Light RE"; Eisai Co.), and the mixture was stirred for 5 minutes. The purified water was added thereto, which was subjected to granulation. The granules were dried on a fluid bed at a suction temperature of 80°C and sieved through a No.20 wire gauze. On the other hand, 2000 g of D-mannitol (Nikken Chemical & Synthetic Ind.; average particle size 50 µm, specific surface area 0.17 m²/g) was placed in a high-speed stirring granulator, and purified water was added thereto, which was subjected to granulation, and the obtained granules were dried on a fluid bed and sieved with a No.20 wire gauze.

PVS/calcium silicate-containing granules (148 g) was mixed with 948 g of the D-mannitol granules, 74 g of crospovidone, 18 g of aspartame, 12 g of magnesium stearate and 1 g of mint flavor to yield granules for tableting. Using a rotary-type tablet machine (HATA-AP15; Hata Iron Works) equipped with a punch of 7 mmφ flat type, the granules were subjected to compression molding under pressure of 700 kg to yield tablets of 120 mg/tablet.

### Example 2

In a high-speed stirring granulator was placed 500 g of PVS and 240 g of calcium silicate ("Flow Light RE"; Eisai Co.); and the mixture was stirred for 5 minutes. The purified water was added thereto, which was subjected to granulation. The granules were dried on a fluid bed at a suction temperature of 80°C and sieved through a No.20 wire gauze. On the other hand, 2000 g of D-mannitol (Nikken Chemical & Synthetic Ind.; average particle size 50 µm, specific surface area 0.17 m²/g) was placed in a high-speed stirring granulator, and purified water was added thereto, which was subjected to granulation, and the obtainedgranules were dried on a fluid bed and sieved with a No.20 wire gauze.

PVS/calcium silicate-containing granules (148 g) was mixed with 960 g of the D-mannitol granules and 74 g of crospovidone to yield granules for tableting. Using a rotary-type tablet machine (HATA-AP15; Hata Iron Works; equipped with a lubricant-spraying apparatus) equipped with apunch of 7 mmφ flat type and an apparatus for spraying a lubricant by pulsating wave, to which had been applied magnesium stearate on the surface of the punch and the die wall, the granules were subjected to compression molding to yield tablets. The molding condition was: tablet weight 120 mg and compression molding under pressure of 700 kg.

### Example 3

Tablets were prepared in the same manner as in Example 2, except that light anhydrous silicic acid (Freund Sangyo; "Adosolider 101") was used in place of calcium silicate.

### Example 4

In a high-speed stirring granulator (Powrex; Type VG-25) was placed 200 g of PVS, 1896 g of D-mannitol (Nikken Chemical & Synthetic Ind.; average particle size 50 µm, specific surface area 0.17m²/g), 96 g of calcium silicate and 148 g of crospovidone, and the mixture was stirred for 5 minutes. Purified water was added thereto, whichwas subjected to granulation. The granules were dried on a fluid bed at a suction temperature of 80°C and sieved through a No.20 wire gauze. To the sieved granules (1170 g) was added 18 g of aspartame, 12 g of magnesium stearate and 1 g of mint flavor to give granules for tableting. Using a rotary-type tablet machine (HATA-AP15; Hata Iron Works) equipped with a punch of 7 mmφ flat type, the granules were subjected to compression molding under pressure of 700 kg to yield tablets of 120 mg/tablet.

### Example 5

In a high-speed stirring granulator was placed 500 g of calcium silicate ("Flow Light RE"; Eisai Co.), a solution of 500 g of PVS in 1000 g of purified water was added thereto, which was subjected to granulation. Then, the granules were dried on a fluid bed at a suction temperature of 80°C and sieved through a No.20 wire gauze. On the other hand, 2000 g of D-mannitol (Nikken Chemical & Synthetic Ind.; average particle size 50 µm, specific surface area 0.17 m²/g) was placed in a high-speed stirring granulator, and purified water was added thereto, which was subjected to granulation, and the obtained granules were dried on a fluid bed and sieved with a No.20 wire gauze.

PVS/calcium silicate-containing granules (200 g) were mixed with 908 g of the D-mannitol granules, 74 g of crospovidone, 18 g of aspartame and 1 g of mint flavor to yield granules for tableting. Using a rotary-type tabletmachine (HATA-AP15 ; Hata Iron Works; equipped with a lubricant-spraying apparatus) equipped with a punch of 7 mmφ flat type and an apparatus for spraying a lubricant by pulsating wave, to which had been applied magnesium stearate on the surface of the punch and the die wall, the granules were subjected to compression molding to yield tablets. The molding condition was: tablet weight 120 mg and compression molding under pressure of 700 kg.

### Example 6

In a high-speed stirring granulator was placed 500 g of PVS and 240 g of calcium silicate ("Flow Light RE"; Eisai Co.), and the mixture was stirred for 5 minutes. Purified water was added thereto, which was subjected to granulation. Then, the granules were dried on a fluid bed at a suction temperature of 80°C and sieved through a No.20 wire gauze. On the other hand, 2000 g of D-mannitol (Nikken Chemical & Synthetic Ind.; average particle size 30 µm, specific surface area 0.40 m²/g) was placed in a high-speed stirring granulator, and purified water was added thereto, which was subject and granulated with subjected to granulation, and the obtained granules were dried on a fluid bed and sieved with a No.20 wire gauze.

PVS/calcium silicate-containing granules (148 g) was mixed with 960 g of the D-mannitol granules and 74 g of crospovidone to yield granules for tableting. Using a rotary-type tablet machine (HATA-AP15; Hata Iron Works; equipped with a lubricant-spraying apparatus) equipped with a punch of 7 mmφ flat type and an apparatus for spraying a lubricant by pulsating wave, to which had been applied magnesium stearate on the surface of the punch and the die wall, the granules were subjected to compression molding to yield tablets. The molding condition was: tablet weight 120 mg and compression molding under pressure of 700 kg.

### Example 7

In a high-speed stirring granulator was placed 500 g of PVS and 1000 g of calcium silicate ("Flow Light RE"; Eisai Co.), and the mixture was stirred for 5 minutes. Purified water was added thereto, which was subjected to granulation. Then, the granules were dried on a fluid bed at a suction temperature of 80°C and sieved through a No.20 wire gauze. On the other hand, 2000 g of D-mannitol (Nikken Chemical & Synthetic Ind.; average particle size 50 µm, specific surface area 0.17 m²/g) was placed in a high-speed stirring granulator, and purified water was added thereto, which was subjected to granulation, and the obtained granules were dried on a fluid bed and sieved with a No.20 wire gauze.

PVS/calcium silicate-containing granules (300 g) were mixed with 820 g of the D-mannitol granules and 80 g of crospovidone to yield granules for tableting. Using a rotary-type tablet machine (HATA-AP15; Hata Iron Works; equipped with a lubricant-spraying apparatus) equipped with a punch of 7mmφ flat type and an apparatus for spraying a lubricant by pulsating wave, to which had been applied magnesium stearate on the surface of the punch and the die wall, the granules were subjected to compression molding to yield tablets. The molding condition was: tablet weight 120 mg and compression molding under pressure of 700 kg.

### Test Example 1

For the tablets prepared in Comparative Example 1 and Examples 1 to 7, the time required for disintegration in the oral cavity and the hardness were measured.

As to the disintegration time in the oral cavity, 5 healthy adults held one tablet in his mouth, and the time required for disintegration with his saliva alone was measured and the average value was calculated. As to the hardness of tablets, each of 5 tablets was subjected to the measurement using a tablet-hardness indicator (Toyama Chemical Co., Type TH-303) to calculate the mean value. Table 1 shows the results.

**Table 1**

| | Disintegration time | Hardness |
|---|---|---|
| Comparative Ex.1 | 87 sec. | 33N |
| Example 1 | 21 sec. | 28N |
| Example 2 | 10 sec. | 35N |
| Example 3 | 15 sec. | 32N |
| Example 4 | 28 sec. | 36N |
| Example 5 | 14 sec. | 35N |
| Example 6 | 15 sec. | 42N |
| Example 7 | 13 sec. | 36N |

The tablets of the present invention show the hardness of more than 20N, and the disintegration time was less than 30 seconds.

### Industrial Applicability

The present invention provides intraorally rapidly disintegrable tablets which rapidly disintegrate in the oral cavity without water and hold practical hardness to cause no problem such as losing its shape in the course of production or during distribution as well as in handling in a hospital or by a patient. The intraorally rapidly disintegrable tablets can easily be taken at an accurate dose by patients including aged persons or infants whose swallowing ability is weak. Moreover, patients other than the aged or infants can also receive the tablets without water anywhere in the outdoors wherein no water is available. Thus, the tablets of the present invention are advantageous in improving the medication compliance for all of the patients.

## Claims

1. An intraorally rapidly disintegrable tablet which comprises fine granules prepared by granulating a mixture of a water-soluble pharmacologically active ingredient and an adsorbent, D-mannitol and a disintegrator.

2. The intraorally rapidly disintegrable tablet as claimed in Claim 1, wherein the adsorbent is at least one member selected from the group consisting of calcium silicate, light anhydrous silicic acid, synthetic aluminum silicate, silicon dioxide and magnesium metasilicate aluminate.

3. The intraorally rapidly disintegrable tablet as claimed in Claim 1 or 2, wherein the disintegrator is at least one member selected from the group consisting of crospovidone, low-substituted hydroxypropylcellulose, croscarmellose sodium and carboxymethylcellulose.

4. The intraorally rapidly disintegrable tablet as claimed in any one of Claims 1 to 3, wherein the whole or a part of the D-mannitol is a primary particle and the specific surface area of the primary particle is 1.0 m²/g or less.

5. The intraorally rapidly disintegrable tablet as claimed in any one of Claims 1 to 4, wherein the solubility of the water-soluble pharmacologically active ingredient in water is 1 mg/ml or more.

6. The intraorally rapidly disintegrable tablet as claimed in any one of Claims 1 to 4, wherein the water-soluble pharmacologically active ingredient is pravastatin sodium.

7. The intraorally rapidly disintegrable tablet as claimed in any one of Claims 1 to 6, further containing a lubricant.

8. The intraorally rapidly disintegrable tablet as claimed in Claim 7, wherein the lubricant is contained only on the surface of the tablet.

9. The intraorally rapidly disintegrable tablet as claimed in Claim 7 or 8, wherein the lubricant is at least one member selected from the group consisting of magnesium stearate, calcium stearate, stearic acid, stearyl alcohol, sodium stearyl fumarate, sucrose fatty acid ester and talc.

10. The intraorally rapidly disintegrable tablet as claimed in any one of Claims 1 to 9, further containing at least one member selected from the group consisting of flavoring agents, sweeteners, perfumes, coloring agents, stabilizers, fluidizing agents, anti-oxidants and co-solubilizers.

11. The intraorally rapidly disintegrable tablet as claimed in any one of Claims 1 to 10, wherein the compounding ratio of the water-soluble pharmacologically active ingredient to the adsorbent in the fine granules is 1:10 to 10:1.

12. The intraorally rapidly disintegrable tablet as claimed in any one of Claims 1 to 11, wherein the compounding ratio of the fine granules in the tablet is 1 to 50% by weight.

13. The intraorally rapidly disintegrable tablet as claimed in any one of Claims 1 to 12, wherein the compounding ratio of the D-mannitol in the tablet is 20 to 99% by weight.

14. The intraorally rapidly disintegrable tablet as claimed in any one of Claims 1 to 13, wherein the compounding ratio of the disintegrator in the tablet is 0.5 to 30% by weight.

15. The intraorally rapidly disintegrable tablet as claimed in any one of Claims 1 to 14, wherein the hardness of the tablet is 20N or higher.

16. The intraorally rapidly disintegrable tablet as claimed in any one of Claims 1 to 15, wherein the disintegration time in oral cavity is 30 seconds or less.

17. A process for producing an intraorally rapidly disintegrable tablet which comprises mixing fine granules prepared by granulating a mixture of a water-soluble pharmacologically active ingredient and an adsorbent, D-mannitol and a disintegrator to prepare a material for compression molding, and subjecting the material to compression molding.

18. The process as claimed in Claim 17, wherein the adsorbent is at least one member selected from the group consisting of calcium silicate, light anhydrous silicic acid, synthetic aluminum silicate, silicon dioxide and magnesium metasilicate aluminate.

19. The process as claimed in Claim 17 or 18, wherein the disintegrator is at least one member selected from the group consisting of crospovidone, low-substituted hydroxypropylcellulose, croscarmellose sodium and carboxymethylcellulose.

20. The process as claimed in any one of Claims 17 to 19, wherein the whole or a part of the D-mannitol is a primary particle and the specific surface area of the primary particle is 1.0 m²/g or less.

21. The process as claimed in any one of Claims 17 to 20, wherein the solubility of the water-soluble pharmacologically active ingredient in water is 1 mg/ml or more.

22. The process as claimed in any one of Claims 17 to 20, wherein the water-soluble pharmacologically active ingredient is pravastatin sodium.

23. The process as claimed in any one of Claims 17 to 22, wherein the material for compression molding contains a lubricant.

24. The process as claimed in any one of Claims 17 to 23, wherein the compression molding is carried out using a compression molding machine in which a lubricant is previously applied on the surface of punch and the die.

25. The process as claimed in Claim 23 or 24, wherein the lubricant is at least one member selected from the group consisting of magnesium stearate, calcium stearate, stearic acid, stearyl alcohol, sodium stearyl fumarate, sucrose fatty acid ester and talc.

26. The process as claimed in any one of Claims 17 to 25, wherein the material for compression molding contains at least one member selected from the group consisting of flavoring agents, sweeteners, perfumes, coloring agents, stabilizers, fluidizing agents, anti-oxidants and co-solubilizers.

27. The process as claimed in any one of Claims 17 to 26, wherein the compounding ratio of the water-soluble pharmacologically active ingredient to the adsorbent in the fine granules is 1:10 to 10:1.

28. The process as claimed in any one of Claims 17 to 27, wherein the compounding ratio of the fine granules in the tablet is 1 to 50% by weight.

29. The process as claimed in any one of Claims 17 to 28, wherein the compounding ratio of the D-mannitol in the tablet is 20 to 99% by weight.

30. The process as claimed in any one of Claims 17 to 29, wherein the compounding ratio of the disintegrator in the tablet is 0.5 to 30% by weight.

31. An intraorally rapidly disintegrable tablet which is produced by mixing fine granules prepared by granulating a mixture of a water-soluble pharmacologically active ingredient and an adsorbent D-mannitol and a disintegrator to prepare a material for compression molding and subjecting the material to compression molding.

32. The intraorally rapidly disintegrable tablet as claimed in Claim 31, wherein the adsorbent is at least one member selected from the group consisting of calcium silicate, light anhydrous silicic acid, synthetic aluminum silicate, silicon dioxide and magnesium metasilicate aluminate.

33. The intraorally rapidly disintegrable tablet as claimed in any one of Claim 31 or 32, wherein the disintegrator is at least one member selected from the group consisting of crospovidone, low-substituted hydroxypropylcellulose, croscarmellose sodium and carboxymethylcellulose.

34. The intraorally rapidly disintegrable tablet as claimed in any one of Claims 31 to 33, wherein whole or a part of the D-mannitol is a primary particles and the specific surface area of the primary particle is 1.0 m²/g or less.

35. The intraorally rapidly disintegrable tablet as claimed in any one of Claims 31 to 34, wherein the solubility of the water-soluble pharmacologically active ingredient in water is 1 mg/ml or more.

36. The intraorally rapidly disintegrable tablet as claimed in any one of Claims 31 to 34, wherein the water-soluble pharmacologically active ingredient is pravastatin sodium.

37. The intraorally rapidly disintegrable tablet as claimed in any one of Claims 31 to 36,wherein the material for compression molding contains a lubricant.

38. The intraorally rapidly disintegrable tablet as claimed in any one of Claims 31 to 37,wherein the compression molding is carried out using a compression molding machine in which a lubricant is previously applied on the surface of punch and the die.

39. The intraorally rapidly disintegrable tablet as claimed in Claim 37 or 38, wherein the lubricant is at least one member selected from the group consisting of magnesium stearate, calcium stearate, stearic acid, stearyl alcohol, sodium stearyl fumarate, sucrose fatty acid ester and talc.

40. The intraorally rapidly disintegrable tablet as claimed in any one of Claims 31 to 39, wherein the material for compression molding contains at least one member selected from the group consisting of flavoring agents, sweeteners, perfumes, coloring agents, stabilizers, fluidizing agents, anti-oxidants and co-solubilizers.

41. The intraorally rapidly disintegrable tablet as claimed in any one of Claims 31 to 40, wherein the compounding ratio of the water-soluble pharmacologically active ingredient to the adsorbent in the fine granules is 1:10 to 10:1.

42. The intraorally rapidly disintegrable tablet as claimed in any one of Claims 31 to 41, wherein the compounding ratio of the fine granules in the tablet is 1 to 50% by weight.

43. The intraorally rapidly disintegrable tablet as claimed in any one of Claims 31 to 42, wherein the compounding ratio of the D-mannitol in the tablet is 20 to 99% by weight.

44. The intraorally rapidly disintegrable tablet as claimed in any one of Claims 31 to 43, wherein the compounding ratio of the disintegrator in the tablet is 0.5 to 30% by weight.

45. The intraorally rapidly disintegrable tablet as claimed in any one of Claims 31 to 44, wherein the hardness of the tablet is 20N or higher.

46. The intraorally rapidly disintegrable tablet as claimed in any one of Claims 31 to 45, wherein the disintegration time in oral cavity is 30 seconds or less.

47. A process for producing an intraorally rapidly disintegrable tablet which comprises granulating a mixture of a water-soluble pharmacologically active ingredient, an adsorbent, D-mannitol and a disintegrator to prepare a material for compression molding, and subjecting the material to compression molding.

48. The process as claimed in Claim 47, wherein the adsorbent is at least one member selected from the group consisting of calcium silicate, light anhydrous silicic acid, synthetic aluminum silicate, silicon dioxide and magnesium metasilicate aluminate.

49. The process as claimed in Claim 47 or 48, wherein the disintegrator is at least one member selected from the group consisting of crospovidone, low-substituted hydroxypropylcellulose, croscarmellose sodium and carboxymethylcellulose.

50. The process as claimed in any one of Claims 47 to 49, whereinwhole or a part of the D-mannitol is a primary particle and the specific surface area of the primary particle is 1.0 m²/g or less.

51. The process as claimed in any one of Claims 47 to 50, wherein the solubility of the water-soluble pharmacologically active ingredient in water is 1 mg/ml or more.

52. The process as claimed in any one of Claims 47 to 50, wherein the water-soluble pharmacologically active ingredient is pravastatin sodium.

53. The process as claimed in any one of Claims 47 to 52, wherein the material for compression molding contains a lubricant.

54. The process as claimed in any one of Claims 47 to 53, wherein the compression molding is carried out using a compression molding machine in which a lubricant is previously applied on the surface of punch and the die.

55. The process as claimed in Claim 53 or 54, wherein the lubricant is at least one member selected from the group consisting of magnesium stearate, calcium stearate, stearic acid, stearyl alcohol, sodium stearyl fumarate, sucrose fatty acid ester and talc.

56. The process as claimed in any one of Claims 47 to 55, wherein the compression molding material containis at least one member selected from the group consisting of flavoring agents, sweeteners, perfumes, coloring agents, stabilizers, fluidizing agents, anti-oxidants and co-solubilizers.

57. The process as claimed in any one of Claims 47 to 56, wherein the compounding ratio of the water-soluble pharmacologically active ingredient to the adsorbent in the compression molding material is 1:10 to 10:1.

58. The process as claimed in any one of Claims 47 to 57, wherein the compounding ratio of the water-soluble pharmacologically active ingredient and the adsorbent in the tablet is 1 to 50% by weight.

59. The process as claimed in any one of Claims 47 to 58, wherein the compounding ratio of the D-mannitol in the tablet is 20 to 99% by weight.

60. The process as claimed in any one of Claims 47 to 59, wherein the compounding ratio of the disintegrator in the tablet is 0.5 to 30% by weight.

61. An intraorally rapidly disintegrable tablet which comprises awater-soluble pharmacologically active ingredient, an adsorbent, D-mannitol and a disintegrator.

62. The intraorally rapidly disintegrable tablet as claimed in Claim 61, wherein the adsorbent is at least one member selected from the group consisting of calcium silicate, light anhydrous silicic acid, synthetic aluminum silicate, silicon dioxide and magnesium metasilicate aluminate.

63. The intraorally rapidly disintegrable tablet as claimed in Claim 61 or 62, wherein the disintegrator is at least one member selected from the group consisting of crospovidone, low-substituted hydroxypropylcellulose, croscarmellose sodium and carboxymethylcellulose.

64. The intraorally rapidly disintegrable tablet as claimed in any one of Claims 61 to 63, wherein whole or a part of the D-mannitol is a primary particle and the specific surface area of the primary particle is 1.0 m²/g or less.

65. The intraorally rapidly disintegrable tablet as claimed in any one of Claims 61 to 64, wherein the solubility of the water-soluble pharmacologically active ingredient in water is 1 mg/ml or more.

66. The intraorally rapidly disintegrable tablet as claimed in any one of Claims 61 to 64, wherein the water-soluble pharmacologically active ingredient is pravastatin sodium.

67. The intraorally rapidly disintegrable tablet as claimed in any one of Claims 61 to 66, containing a lubricant.

68. The intraorally rapidly disintegrable tablet as claimed in Claim 67, wherein the lubricant is contained only on the surface of the tablet.

69. The intraorally rapidly disintegrable tablet as claimed in Claim 67 or 68, wherein the lubricant is at least one member selected from the group consisting of magnesium stearate, calcium stearate, stearic acid, stearyl alcohol, sodium stearyl fumarate, sucrose fatty acid ester and talc.

70. The intraorally rapidly disintegrable tablet as claimed in any one of Claims 61 to 69, further containing at least one member selected from the group consisting of flavoring agents, sweeteners, perfumes, coloring agents, stabilizers, fluidizing agents, anti-oxidants and co-solubilizers.

71. The intraorally rapidly disintegrable tablet as claimed in any one of Claims 61 to 70, wherein the compounding ratio of the water-soluble pharmacologically active ingredient to the adsorbent is 1:10 to 10:1.

72. The intraorally rapidly disintegrable tablet as claimed in any one of Claims 61 to 71, wherein the compounding ratio of the water-soluble pharmacologically active ingredient and the adsorbent in the tablet is 1 to 50% by weight.

73. The intraorally rapidly disintegrable tablet as claimed in any one of Claims 61 to 72, wherein the compounding ratio of the D-mannitol in the tablet is 20 to 99% by weight.

74. The intraorally rapidly disintegrable tablet as claimed in any one of Claims 61 to 73, wherein the compounding ratio of the disintegrator in the tablet is 0.5 to 30% by weight.

75. The intraorally rapidly disintegrable tablet as claimed in any one of Claims 61 to 74, wherein the hardness of the tablet is 20N or higher.

76. The intraorally rapidly disintegrable tablet as claimed in any one of Claims 61 to 75, wherein the disintegration time in the oral cavity is 30 seconds or less.
